# EUROPEAN PATENT SPECIFICATION

(11) **EP 0 798 002 B1**
(45) Date of publication and mention of the grant of the patent: **24.04.2002**
(21) Application number: 97106298.9
(22) Date of filing: 17.01.1992
(51) Int. Cl.: A61K 39/395

(54) **Anti platelet derived growth factor antibodies in the treatment of restenosis**
Anti-Plättchen-Wachstumsfaktor-Antikörper in der Behandlung von Restenose
Anticorps contre le facteur de croissance dérivé de plaquettes dans le traitement de la resténose

(30) Priority: 17.01.1991 US 641758
(43) Date of publication of application: 01.10.1997
(62) Divisional of application: 92905617.4
(73) Proprietor: THE BOARD OF REGENTS OF THE UNIVERSITY OF WASHINGTON, Seattle, WA 98105 (US)
(72) Inventor: Ross, Russell, Seattle, Washington 98105 (US); Reidy, Michael A., Seattle, Washington 98115 (US); Raines, Elaine W., Seattle, Washington 98146 (US); Lindner, Volkhard, Seattle, WA 98117 (US); Ferns, Gordon A.A., Brentwood, Essex CM14 4SU (GB); Jackson, Christopher, Sandwich, Kent CZ13 9JR (GB)
(74) Representative: Grünecker, Kinkeldey, Stockmair & Schwanhäusser Anwaltssozietät

(56) References cited:
- US-A-7365715 (W. LA ROCHELLE ET AL) 23 JANUARY 1990 (NTIS GAZETTE) XP002035385
- BIOLOGICAL ABSTRACTS, vol. 91, no. 5, 1991 Philadelphia, PA, US; abstract no. 47365, IKEDA U. ET AL: "Mitogenic action of interleukin 1-alpha on vascular smooth muscle cells mediated by PDGF" XP002035386 & ATHEROSCLEROSIS 84(2/3): 183-188, 1990
- SCIENCE, vol. 243, 1989, pages 393-396, XP002035384 RAINES E.W. ET AL: "Interleukin-1 mitogenic activity for fibroblasts and smooth muscle cells is due to PDGF-AA"
- SCIENCE, vol. 253, September 1991, LANCASTER, PA US, pages 1129-1132, XP000674610 FERNS, G.A.A. ET AL: "Inhibition of neointimal smooth muscle acumulation after angioplasty by an antibody to PDGF"
- SCIENCE, vol. 248, 1990, LANCASTER, PA US, pages 1009-1012, XP000674609 ROSS, R. ET AL: "Localization of PDGF-B protein in macrophages in all phases of atherogenesis"

## Description

### Technical Field

The present invention relates to a use of an anti-PDGF antibody as a medicament for inhibiting restenosis in a mammal following angioplasty, endarterectomy and bypass surgery.

### Background of the Invention

Proliferation of smooth muscle cells (SMCs) in the vessel wall is an important event in the formation of vascular lesions in atherosclerosis or in response to vascular injury. Treatment of atherosclerosis frequently includes the clearing of blocked vessels by angioplasty, endarterectomy or bypass surgery, surgical procedures in which atherosclerotic plaques are compressed or removed through catheterization (angioplasty) or stripped away from the arterial wall through an incision (endarterectomy ) or bypassed by anastomising a vein or artery proximal or distal to the site of occlusion (bypass). These procedures remove the vascular endothelium, disturb the underlying intimal layer, and result in the death of medial SMCs. This injury is followed by medial SMC proliferation and migration into the intima, which characteristically occurs within the first few weeks after injury and stops when the overlying endothelial layer is reestablished.

In 30%-40% or more of patients treated by angioplasty, endarterectomy or bypass surgery, thrombosis and/or SMC proliferation in the intima causes re-occlusion of the vessel and consequent failure of the angioplasty, endartarectomy or bypass procedure. This closure of the vessel subsequent to surgery is known as restenosis.

A similar process of SMC proliferation has also been observed in vascular grafts at the perianastomotic site where the graft is surgically joined to the artery wall, as well as with organ transplants, and may contribute to transplant rejection.

It has been postulated that growth factors, such as platelet derived growth factor (PDGF), play a role in the development of atherosclerotic plaques (reviewed by Ross et al., Cell 46: 155-169, 1986). PDGF has been detected within macrophages in all stages of lesion development in both human and nonhuman primate atherosclerosis (Ross et al., Science 248:1009-1012, 1990). One proposed mechanism for plaque formation is the release by platelets, at sites of endothelial denudation, of growth factors that stimulate SMC growth (Ross and Glomset, N. Eng. J. Med. 295: 369-377, 420-425, 1976; Ross, Arteriosclerosis 1: 293-311, 1981). Moore et al. (Thrombos. Haemostas. (Stuttg.) 35: 70, 1976) and Friedman et al. (J. Clin. Invest. 60: 1191-1201, 1977), using an indwelling catheter injury model, reported an inhibition of experimentally induced intimal lesion formation in rabbit arteries by prolonged thrombocytopenia induced by administration of anti-platelet serum. It has also been postulated that SMCs may themselves produce PDGF which stimulates lesion development through an autocrine mechanism (Ross et al., ibid; Walker et al., Proc. Natl. Acad. Sci. USA 83: 7311-7315, 1986). Fingerle et al. (Proc. Natl. Acad. Sci. USA 86: 8412-8416, 1989) investigated intimal lesion formation in thrombocytopenic rats and concluded that platelets do not play a role in the initial SMC proliferation after balloon injury but may regulate SMC migration into the intima. Platelets are now known to release a number of growth factors, including PDGF, transforming growth factors alpha and beta (TGFα and TGFβ), insulin-like growth factor I (IGF-I) and platelet derived endothelial cell growth factor. However, there has been no direct evidence to demonstrate that a particular mitogen or mitogens is responsible for the development of arterial lesions.

US-A-7365715 describes the preparation of neutralizing monoclonal antibodies to human platelet derived growth factor (PDGF), and proposes different therapeutical applications of the anti-PDGF antibodies, among others for the treatment of acute arteriole stenosis. In Biological Abstracts, vol. 91, no. 5, 1991, abstract no. 47365, the authors report that antibodies specific for PDGF totally inhibit the interleukin-1 (IL-1) induced thymidine uptake, and conclude that IL-1 promotes the growth of of vascular smooth muscle cells (VSMC) via induction of endogenous PDGF production and might thus participate in the abnormal proliferation of VSCM that occur early in atherogenesis. Raines et al. (Science, vol. 243, 1989, 393-6) report that both IL-1 and PDGF are able to induce proliferation of connective tissue cells *in vivo.*

Removal of atherosclerotic plaques by angioplasty, endartarectomy or treatment by bypass surgery has limited efficacy, and no effective treatment for restenosis has been developed. There is therefore a need in the art for methods of inhibiting restenosis following angioplasty, endarterectomy and bypass surgery in a mammal.

The present invention provides such methods and fulfills other, related needs.

### Disclosure of the Invention

The above object is achieved by the use of an anti-PDGF antibody for preparing a medicament for inhibiting restenosis in a mammal following angioplasty, endarterectomy and bypass surgery. Preferably, the anti-PDGF antibody for use according to the invention is a monoclonal antibody.

In one embodiment, the antibody is administered prior to angioplasty, endarterectomy or bypass surgery. In another embodiment, the antibody is administered subsequent to angioplasty, endarterectomy or bypass surgery. In yet another embodiment, a panel of anti-growth factor antibodies is used, such as a panel of antibodies capable of neutralizing the AA, AB and BB isoforms of platelet derived growth factor.

These and other aspects of the invention will become evident upon reference to the following detailed description and the attached drawings.

### Brief Description of the Drawings

Figure 1 is a graph illustrating the effect of goat anti-PDGF on PDGF activity in rat whole blood serum. The results are expressed as the mean +/- SEM for triplicate determinations.

Figure 2 is a graph illustrating the anti-PDGF IgG inhibition of the chemotactic response of rat platelet releasate. Samples are identified as follows: (1), PDGF-AA (5 ng/ml); (2), PDGF-BB (10 ng/ml); (3), TGF-β (300 pg/ml); (4), bFGF (500 pg/ml); (5), rat platelet releasate (RPR); (6), RPR plus 500 ug of anti-PDGF per milliliter; (7), RPR plus 1 mg of anti-PDGF per milliliter; (8), nonimmune IgG (1 mg/ml); and (9), control media. The results are expressed as fold increase (mean +/- SEM) in cell migration above a buffer control.

Figure 3 is a pair of photographs comparatively illustrating the inhibition of the accumulation of intimal smooth muscle cells 8 days after balloon catheter injury. Panel A represents treatment with nonimmune goat IgG; Panel B represents treatment with anti-PDGF IgG.

Figure 4 is a graph illustrating the effect of nonimmune IgG and anti-PDGF IgG on the accumulation of intimal smooth muscle cells after balloon catheter injury. Measurements were made on intimal cross-sectional areas and the results expressed as, the mean +/- SEM.

Figure 5 is a graph illustrating the clearance of anti-PDGF IgG from the plasma of a nude rat after administration of a single intra-peritoneal injection (60 mg/100g body weight).

Figures 6A, 6B and 6C illustrate the effects of anti-PDGF on the intimal and medial response to a filament loop injury of the right carotid artery of a nude rat. Figure 6A depicts the effect on the intima-media cross-sectional area; Figure 6B, the effect on intimal cellularity; and Figure 6C, the effect on intimal and medial proliferation at 8 days after injury measured by H-thymidine incorporation and autoradiography.

### Detailed Description of the Invention

As noted above, restenosis of blood vessels is a common problem in patients who have undergone angioplasty, endartarectomy or arterial bypass surgery. Restenosis is believed to proceed via a process that includes both proliferation (mitosis) and migration of vascular smooth muscle cells in the area damaged by the surgical procedure.

Antibodies useful within the present invention may be produced by conventional procedures of immunization and purification. Briefly, a purified growth factor is administered to an animal such as a mouse, rat, rabbit or goat in an amount sufficient to cause an immune response. It is preferred to administer the growth factor in combination with an adjuvant, such as Freund's adjuvant, in order to enhance the immune response. Although a single injection of growth factor may be sufficient to induce antibody production in the animal, it is generally preferred to administer a large initial injection followed by one or more booster injections over a period of several weeks to several months. See, e.g., Hurrell, J.G.R., ed., Monoclonal Hybridoma Antibodies: Techniques and Applications, CRC Press Inc., Boca Raton, FL, 1982. Blood is then collected from the animal and clotted, and antibodies are isolated from the serum using conventional techniques such as salt precipitation, ion exchange chromatography, affinity chromatography or high performance liquid chromatography. Growth factors for use in immunization are prepared from natural sources or genetically engineered cells according to conventional methods such as those described by Raines and Ross (J. Biol. Chem. 257: 5154-5160, 1982), Antoniades (U.S. Patent No. 4,479,896), Murray et al. (U.S. Patents Nos. 4,801,542, 4,845,075 and 4,889,919), Bohlen et al. (FEBS Lett. 185: 177-181, 1985), Barr (WO 90/05184), Fiddes et al. (WO 87/01728) and Moscatelli et al. (EP 226,181). In the alternative, purified growth factors can be obtained from commercial suppliers (e.g., Genzyme Corp., Boston, MA; Collaborative Research, Bedford, MA).

Within one embodiment of the invention, monoclonal antibodies are used. Monoclonal antibodies provide the advantages of ease of production and lower therapeutic doses as compared to polyclonal antisera, since only antibodies of the desired specificity are used. Methods for producing monoclonal antibodies are well known in the art and are disclosed, for example, by Kohler and Milstein (Nature 256: 495, 1975; Eur. J. Immunol. 6: 511-519, 1976). See also Hurrell, J.G.R., ed., Monoclonal Hybridoma Antibodies: Techniques and Applications, CRC Press Inc., Boca Raton, FL, 1982. As will be appreciated by those skilled in the art, antibody fragments, such as Fab fragments, may also be used.

It is generally preferred to use antibodies that are syngeneic with the patient or that contain syngeneic constant regions. For this reason, genetically engineered antibodies will generally be used in the treatment of humans. Methods for producing recombinant human antibodies or humanized non-human (i.e., chimeric) antibodies are disclosed by Cabilly et al. (U.S. Patent No. 4,816,567), Robinson et al. (WO 87/02671) and Neumaier (WO 90/00616). Briefly, human constant region genes are joined to appropriate human or non-human variable region genes. The joined genes are then transfected into host cells, which are cultured according to conventional procedures. In the alternative, monoclonal antibody producing cells may be transfected with cloned human constant region genes, and chimeric antibody genes generated by homologous recombination. Thus it is possible to assemble monoclonal antibodies with a significant portion of the structure being human, thereby providing antibodies that are more suitable for multiple administrations to human patients.

Within the present invention it is preferred to use neutralizing antibodies. "Neutralizing antibody" is used herein to designate an amount of an antibody sufficient to block essentially all of the biological activity of an antigen in an in vitro test system. Suitable in vitro test systems include, inter alia, mitogenesis assays and receptor binding assays. For example, 200 µg/ml of a polyclonal anti-PDGF IgG described herein is able to block the mitogenic and chemotactic activity of 2 ng/ml of each of the dimeric forms of PDGF. As will be understood by those skilled in the art, the amount of antibody needed to neutralize a given amount of antigen will depend on such factors as antibody specificity and affinity.

Because PDGF is a mixture of the three possible dimer combinations (isoforms) of its component chains (known as A-chain and B-chain), the anti-PDGF antibodies used within the present invention will preferably be a panel of antibodies capable of neutralizing all three isoforms (AA, BB and AB). Monoclonal antibodies are preferred. Methods of making isoform-specific anti-PDGF monoclonal antibodies are disclosed by Hart et al. (US-A-5 094 941 ; Biochemistry 29 : 166-172, 1990). Hybridomas producing isoform-specific anti-PDGF monoclonal antibodies have been deposited with the American Type Culture Collection, Rockville, MD, under accession numbers HB 9610, HB 9611, HB 9612 and HB 9613.

Anti-PDGF antibodies will generally be administered prior to surgery and continuing after surgery at intervals of from several hours to several days over the course of one to two weeks or more. In many cases it will be preferable to administer daily doses during a hospital stay, followed by less frequent bolus injections during a period of outpatient treatment. In the alternative, the patient is treated as described above with anti-PDGF antibody following surgery.

Doses of antibody will be selected on the basis of neutralization criteria as described above. Dosage levels are calculated from neutralization data after determining clearance of antibody from the blood. In general, dosage is selected with the goal of maintaining circulating levels of antibody sufficient to neutralize any released growth factors. In general, doses will be in the range of about 20 µg to 600 mg or more of antibody per kg of patient body weight, preferably about 0.1 mg to 20 mg/kg, more preferably about 1 mg-10 mg/kg. Somewhat higher doses may be required if two or more antibodies are administered in combination.

For use within the present invention, anti-PDGF antibodies are formulated into injectable compositions according to conventional procedures and packaged in sterile containers. The antibodies may be combined with a suitable diluent such as sterile saline or sterile water. The antibody compositions may further contain carriers, stabilizers and excipients such as sugars (e.g., mannitol) or albumin. In the alternative, the antibodies may be provided in lyophilized form and reconstituted in a suitable diluent prior to use. These compositions may be packaged in single or multiple dosage form, for example in sealed ampoules or vials. Packages may contain one antibody, a mixture of antibodies (e.g., anti-PDGF and anti-bFGF) or combinations of individual antibodies in separate containers or compartments.

For inhibition of stenosis in vascular grafts, anti-growth factor antibodies are covalently attached to the graft through their constant regions or incorporated into the graft in slow-release formulations.

The following examples are offered by way of illustration, not by way of limitation.

### EXAMPLES

### EXAMPLE 1

Goat antisera were raised to purified human PDGF (Raines and Ross, ibid). Six injections of approximately 75 µg were given at two-week intervals. The initial injection was in complete Freund's and subsequent injections in incomplete Freund's adjuvant. All injections were given subcutaneously at 10 to 15 sites. The first positive bleed was 3 months following the initial injection. Antibody was obtained by plasmapheresis of the animal to allow frequent collection of large amounts of antibody and to prevent release of endogenous PDGF from platelets. All of the antibody used in these studies was obtained following the additional injection of 350 µg of PDGF which resulted in a 5- 20 fold increase in serum titre. Typical titre of the animal removed the mitogenic activity of 2 ng/ml purified PDGF on 3T3 cells at a plasma dilution of 1:320. The IgG fraction was prepared by 18% sodium sulfate precipitation of plasma followed by DEAE-Sephacel chromatography. Normal goat IgG was prepared by the same procedure. Protein concentration of both preparations was determined by the method of Lowry et al., (J. Biol. Chem. 193: 265, 1951).

Three different methods were used to evaluate the specificity of anti-PDGF antibody; immunoprecipitation of ¹²⁵I-test substances, including insulin, EGF, platelet factor 4, β-thromboglobulin, FGF, TGF-β, and TGF-α; inhibition of the mitogenic activity of test samples on 3T3 cells as described in Example 1 using insulin, EGF, FGF, and IGF-1; and inhibition of PDGF competitive activity to evaluate species specificity, in which a serum concentration which resulted in approximately 75% competition in a PDGF radioreceptor assay (performed essentially as described by Bowen-Pope and Ross, Methods Enzymol. 109: 69-100, 1985) was preincubated with 400 µg/ml anti-PDGF IgG for 1 hour at 37°C prior to addition to the 3T3 cells. Anti-PDGF only immunoprecipitated dimeric forms of PDGF and only neutralized the mitogenic activity of dimeric forms of PDGF. PDGF competitive activity in serum from humans, pigs, dog, horse, mouse, rat, chicken, rabbit and non-human primate were completely neutralized by anti-PDGF. Only PDGF competitive activity in serum from sheep, cows and goat were not neutralized by the anti-PDGF.

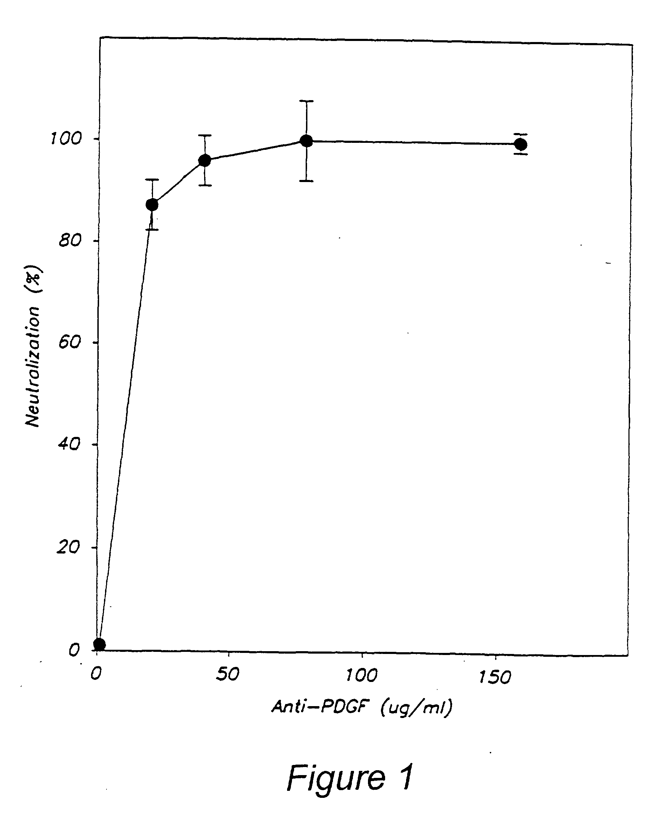

The anti-PDGF completely neutralized the PDGF binding competitive activity present in rat whole blood serum at 50 µg/ml (Figure 1 ). PDGF binding activity in rat whole blood serum (WBS) was assessed by radioreceptor assay with human foreskin fibroblasts (SK5 cells) as the target cell and PDGF-AB as a standard (Bowen-Pope and Ross, Methods Enzymol. 109:69, 1985). A constant amount of rat WBS (25% by volume, which is equivalent to 2.5 ng of PDGF-BB per milliliter) was incubated with increasing concentrations of anti-PDGF before evaluation by radioreceptor assay. The specific binding of ¹²⁵I-labeled PDGF-AB was determined for each sample, and the data were expressed as the percent neutralization of PDGF competitive activity in rat WBS in the absence of anti-PDGF IgG.

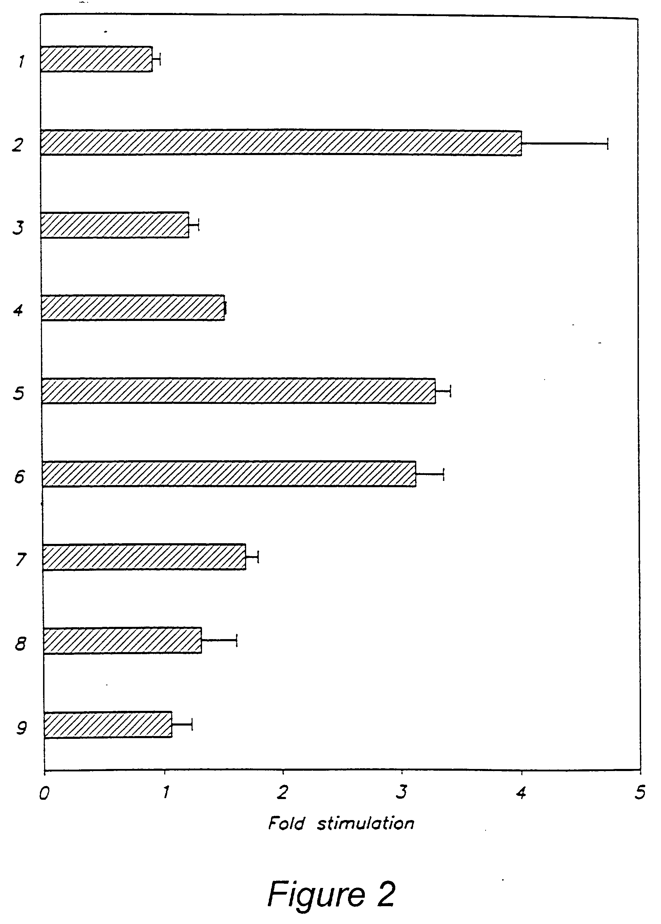

Furthermore, the anti-PDGF substantially prevented PDGF-induced [³H]thymidine incorporation by rat smooth muscle cells and inhibited 50% of the mitogenic activity of rat platelet releasate (Ferns et al., Am. J. Pathol. 138:1045, 1991). The anti-PDGF also inhibited chemotaxis of rat carotid smooth muscle cells to purified PDGF and inhibited most of the chemotactic activity in rat platelet releasate (Figure 2). In contrast, the control medium, the nonimmune IgG, and PDGF-AA exhibited no chemotactic activity (Figure 2).

To determine dosage levels of the antibody in vivo, the clearance of the anti-PDGF was evaluated. Plasma antibody concentrations in rats treated with anti-PDGF determined by enzyme-linked immunosorbent assay (ELISA) were reduced by 50% after 30 hours, and daily intraperitoneal administration of anti-PDGF IgG (60 mg per 100 g of body weight) maintained plasma concentrations of 1000 µg/ml during the 9-day duration of the experiment. At these concentrations, there was no significant effect on platelet counts or complement levels and in vitro chemotaxis to rat platelet releasate was completely inhibited (Figure 2).

Homozygous nude rats aged 4-5 months of age (approximately 200 g weight) were obtained from the National Institutes of Health, Bethesda, MD and housed in a pathogen-free facility. Either goat anti-PDGF or non-immune goat IgG were administered daily by IP injection starting the day before surgery. An antibody dose of 60 mg/100 g body weight was sufficient to achieve antibody levels between 1.5 and 2 mg/ml 24 hours after administration. On the day of surgery, animals were anesthetized using ketamine and Rompun, and both common carotid arteries were balloon catheterized using a 2 French embolectomy catheter. Blood samples were taken during the operative procedure and at sacrifice to determine circulating blood levels of antibody in each animal. Animals were sacrificed 8 days after surgery. Seventeen, 9 and 1 hour prior to sacrifice, animals were injected with [³H]-thymidine (50µCi/100gm) to label proliferating intimal and medial cells. At the time of sacrifice, animals were anesthetized with ketamine and Rompun. Blood was taken for antibody levels and the animals injected with Evans Blue. After 10-15 minutes, the jugular veins were isolated for perfusion run-off with a cannula introduced into the abdominal aorta. The animals were injected with a lethal dose of sodium pentobarbital before perfusion with Ringer's lactate and fixation in situ with 4% paraformaldehyde. Both carotids were divided into three segments so that the uniformity of the lesion could be assessed.

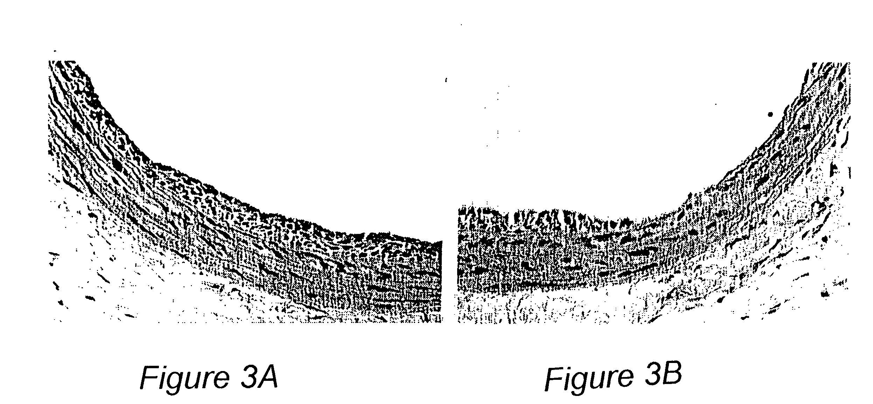

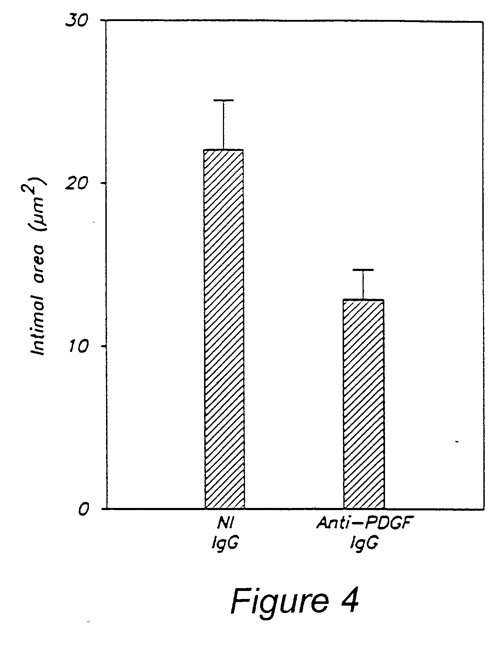

Administration of anti-PDGF before and after balloon catheter deendothelialization reduced the thickness and cellular content of the neointima (Figure 3). Quantitative image analysis of the neointima of the 19 animals in each experimental group demonstrated that administration of anti-PDGF resulted in a 40.9% reduction in the area of the neointima (P < 0.01 by two tailed *t* test) (Figure 4).

### EXAMPLE 2

An anti-human PDGF polyclonal antibody was obtained as generally described in Example 1. Briefly, a goat was immunized with PDGF purified from outdated human platelets (Raines and Ross, ibid.) emulsified in Freund's incomplete adjuvant and administered subcutaneously every two weeks for 3 months. Once a titre was established, plasma was collected by plasmapheresis every week with periodic boosts of PDGF in incomplete Freund's. A concentrated IgG fraction was prepared by sodium sulphate precipitation and DEAE-Sephacel (Pharmacia, Piscataway, NJ) column chromatography. Purified IgG was obtained by elution with 0.01M phosphate buffer (pH 6.8). The pooled void volume and pH 6.8 wash was concentrated by ultrafiltration (PM-10, Amicon Corp., Danvers, MA) and dialyzed against phosphate buffered saline to a protein concentration of approximately 60-90 mg/ml as determined by the method of Lowry et al. (J. Biol. Chem. 193:265, 1951). Bovine serum albumin was used as a standard and a correction factor applied for immunoglobulin (Klosse et al., Clin. Chim. Acta 32:321, 1971). Before use, the IgG was filter-sterilized through a 0.22 µm filter (Millipore, Bedford, MA) and stored at 4°C. A non-immune goat IgG was prepared following the same method, using commercially available goat plasma. The specificity of the antibody was evaluated in the same manner as described in Example 1, with similar results.

Homozygous nude rats (nu/nu) (Ferns et al., Am. J. Path. 138:1045, 1991) were obtained from the breeding colony at the National Institutes of Health (Bethesda, MD), and were maintained in a pathogen-free facility. Animals were administered a single dose of anti-PDGF antibody (60 mg/100 g body weight) i.p. when they were 20-24 weeks of age (250-350 g). Blood samples were then drawn at various times afterwards into tubes containing approximately 0.38% sodium citrate (final concentration). The plasma was separated and stored at -20°C until analyzed by ELISA.

Micro-titre plates (96 well, Nunc) were coated with 10 ng PDGF-AB per well for 18 h. Non-specific protein binding was reduced by blocking with 2% bovine serum albumin (Sigma Chemical Co., St. Louis, MO)/0.2% Tween®-20 in PBS for 1 h at 37°C. Dilutions of standards and samples were prepared in a 1:16 mixture of rat plasma-derived serum and PTB (0.05% Tween® 20/0.2% bovine serum albumin in PBS). One hundred microliter aliquots of sample or standards of known concentrations of anti-PDGF IgG were incubated in wells for 90 min at 37°C. The plates were rinsed 5 times in wash buffer (0.05% Tween® 20/0.9% NaCl), then incubated with 100 µl/well 1:1000 biotinylated anti-goat IgG (Tago Diagnostics, Burlingame, CA) in PTB for 60 min at 37°C. Unbound secondary antibody was removed by rinsing in wash buffer followed by incubation with avidin/biotinylated peroxidase. Substrate (o-phenylenediamine, Sigma Chemical Co.) was dissolved in 0.05M citrate/0.1 M Na₂HPO₄ (pH 5.0) and incubated for 15 min at room temperature. The reaction was terminated by adding 4N sulphuric acid, and the absorbance of the reaction product read at 490 nm.

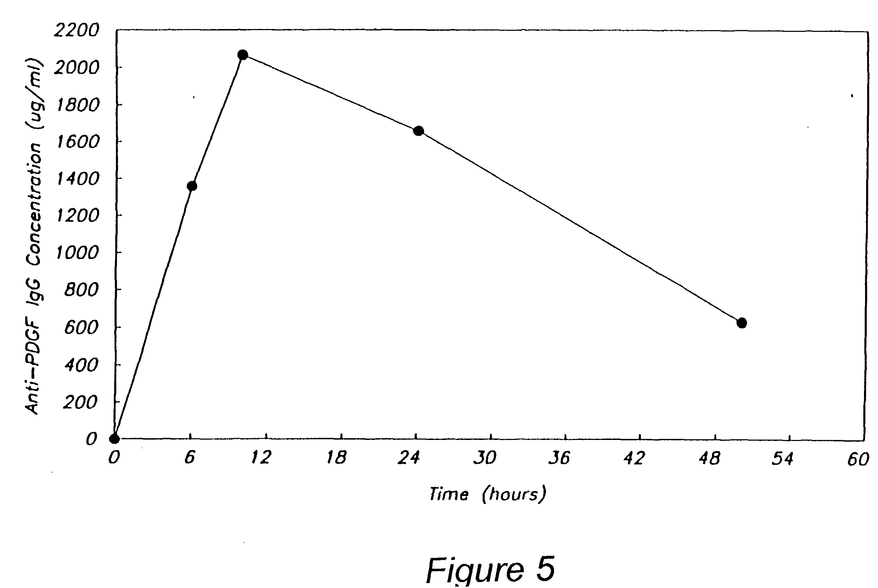

Anti-PDGF IgG injected i.p. had a half life of approximately 24 hours (Figure 5); peak levels were attained within 10 hours after administration. All animals included in the study had plasma levels of anti-PDGF in excess of 1000 µg/ml at the time of carotid injury and sacrifice; most had levels greater than 2000 µg/ml. Platelet counts at the time of surgery and sacrifice were unaffected by anti-PDGF antibody treatment. On the basis of the clearance data, a daily i.p. injection of antibody was found to be adequate to maintain antibody levels at approximately 10-20 times higher than necessary to substantially prevent the effects of PDGF contained in rat serum.

Approximately 18h after a priming dose of antibody, rats were anesthetized with intraperitoneal xylazine (Rompun, Miles Laboratory Inc., Shawnee, KA; 40 mg/kg) and ketamine (Vetalar, Parke-Davis, NJ; 10 mg/kg) and the right carotid bifurcation exposed through a paramedian incision. The endothelium was removed using a nylon filament catheter (Fingerle et al., Arteriosclerosis 10:1082, 1989). A blood sample was obtained perioperatively, anticoagulated with 0.38% sodium citrate and kept on ice until the plasma was separated by centrifugation at 1000 x g and stored at -20°C. This sample was used for the determination of plasma anti-PDGF levels at the time of surgery using the ELISA method. Following balloon catheter de-endothelialization, the skin and deep fascia were closed with metal clips. Each animal then received a further injection of either anti-PDGF (n=19) or non-immune IgG (n=16) post-operatively and then daily until sacrifice. In order to label cells, 17, 9 and 1 h before sacrifice each animal was injected i.p. with 50 uCi/100 g ³H-thymidine (New England Nuclear, Boston, MA).

Eight days post-operatively, animals were sedated with xylazine and ketamine. Both jugular veins were exposed and a paramedian abdominal incision was made to access the aorta for insertion of a cannula. A blood sample was taken from the cannula for determination of plasma anti-PDGF immunoglobulin levels and a platelet count. The cannula was then connected to a perfusion apparatus. The animals were given a lethal dose of sodium pentobarbital, the jugulars were cut and the animals were perfused with Ringer's lactate at a pressure of 120 mm Hg until the run-off was clear. This was then replaced with 4% paraformaldehyde in isotonic PBS (pH 7.4), which was perfused at the same pressure for 15 minutes. Following fixation in-situ, the carotids were isolated and dissected free of adherent fat and connective tissue. Mid-carotid segments were embedded in paraffin.

Five micron sections of carotid were dipped in NTP-2 photographic emulsion (Kodak, Rochester, NY) (Clowes et al., Lab Invest. 49:327, 1983). The autoradiographs were exposed for 14 days at 4°C in a light-tight box and were then developed using Kodak D-19 developer and fixed with Kodak Rapid Fix. Cell nuclei were stained with hematoxylin. Cells with more than five silver grains above the nucleus were considered positive. Duplicate sections from two levels of each carotid were examined under oil immersion using a Zeiss Axioskop microscope with a X100 objective. Approximately 600 cells per wall compartment were counted to determine the proportion of labeled cells.

The intimal and medial cross-sectional areas were measured using an image analysis system consisting of a Leitz microscope fitted with a X25 objective, a digitizing pad and IBM PC with Vids-V software (Ai Cambridge, Papworth, U.K.). Comparisons of the group means were made using the Mann-Whitney U test.

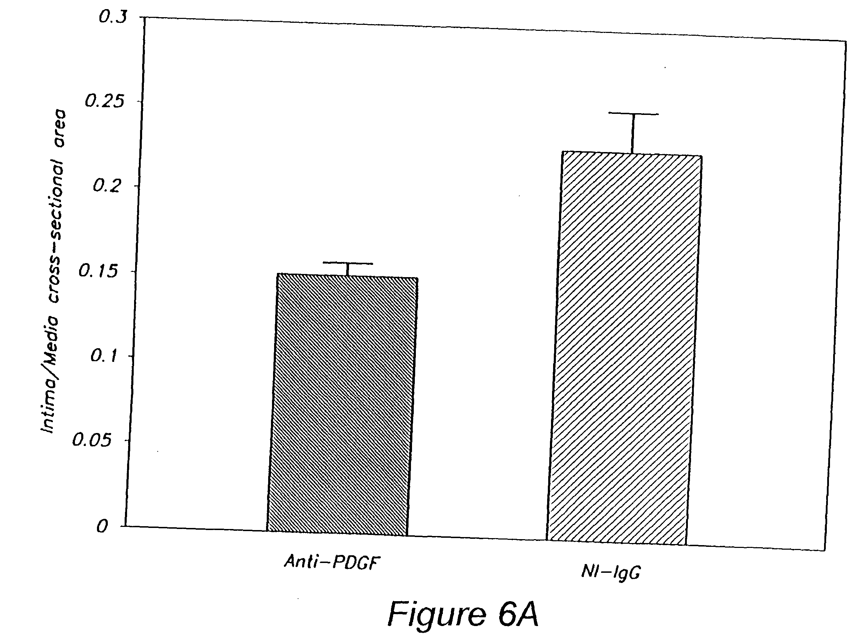

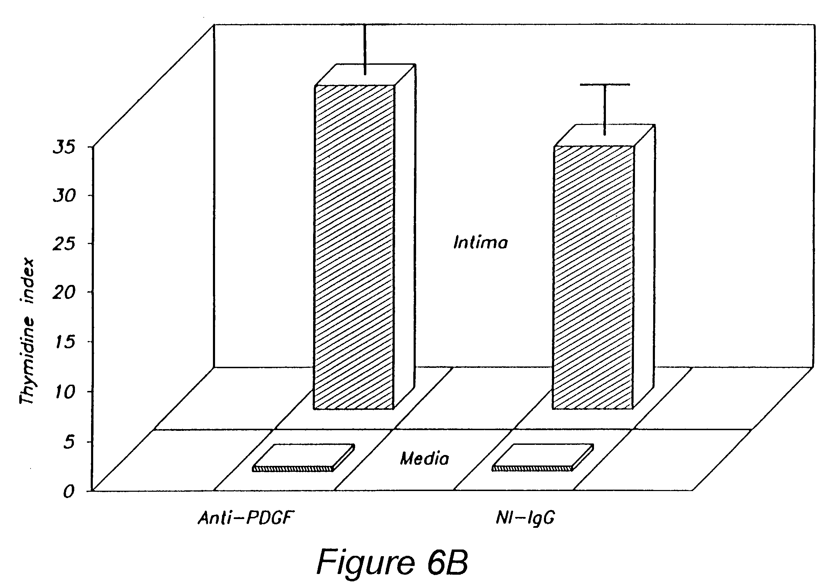

At eight days after injury, carotid injury by the nylon filament loop resulted in a thickened intima which (in the control animals) constituted approximately one fifth the total cross-sectional area of the carotid artery. Neo-intimal cellularity and intima: media cross-sectional area were reduced by 33.2% (p<0.025) and 33.8% (p<0.025) respectively in the anti-PDGF antibody treated animals (Figures 6A and 6B) as compared to controls.

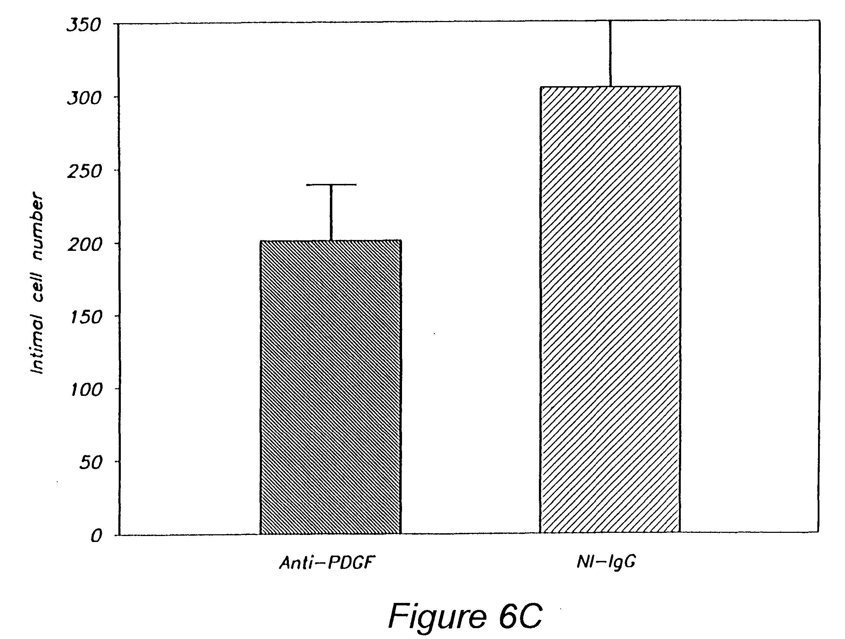

Loop injury induced a marked proliferative response in the neo-intimal cells. Eight days after injury approximately 30% of intimal cells were thymidine labeled. By this time, medial cell proliferation had fallen to approximately 2%. In contrast to its effect on intimal thickening and cellularity, the anti-PDGF antibody had no significant effect on intimal nor medial cell proliferation (Figure 6C).

### EXAMPLE 3

Normal male Sprague-Dawley rats were anesthetized with ether, and were injected intraperitoneally with phosphate-buffered saline (PBS), with normal goat IgG, or with goat anti-PDGF IgG. The dosage volume was 9 ml/kg, and the dose of IgG was 600 mg/kg. The anti-PDGF and the normal IgG were both prepared as described in Examples 1 and 2. Twenty-four hours after the first injection, animals were anesthetized with ketamine/xylazine and both common carotid arteries were injured by 3 passes of a saline-filled, size 2 French balloon catheter. Another injection of PBS or IgG was given immediately after catheterization. Further injections were given under ether anaesthesia 1, 2 and 3 days after catheterization. On the fourth day after catheterization, animals were sacrificed by injection of sodium pentobarbital followed by exsanguination by perfusion with Ringer's buffer. The left common carotid artery was removed for biochemical analysis. The right common carotid artery was perfused at 120 mmHg with Chi's fixative (2% glutaraldehyde, 1% paraformaldehyde in phosphate buffer). This vessel was prepared for en face scanning electron microscopy.

In a separate procedure, normal male Sprague-Dawley rats were anesthetized with ketamine/xylazine, and both common carotid arteries were denuded of endothelium using a filament loop catheter. PBS, rabbit anti-basic FGF IgG, or normal rabbit IgG was injected intravenously in a dosage volume of 1.34 ml/rat: the dose of IgG was 10 mg/rat. The anti-basic FGF IgG and the normal rabbit IgG were both prepared as described in EP-B-0573 510. Five minutes after injection, both common carotid arteries were re-injured by 3 passes of a saline-filled, size 2 French balloon catheter. Further injections of PBS, anti-basic FGF or normal IgG (0.67 ml/rat; 5 mg IgG/rat) were given under ether anaesthesia 1, 2 and 3 days after catheterization. On the fourth day after catheterization, animals were sacrificed by injection of sodium pentobarbital followed by exsanguination by perfusion with Ringer's buffer. The left common carotid artery was removed for biochemical analysis. The right common carotid artery was perfused at 120 mm Hg with Chi's fixative (2% glutaraldehyde, 1% paraformaldehyde in phosphate buffer). This vessel was prepared for en face scanning electron microscopy.

Vessels from the above procedures were opened longitudinally and were pinned out on Teflon cards. They were dehydrated through an ethanol series, and then were dried at the critical point of carbon dioxide in a critical point drier. The dried specimens were mounted on aluminum stubs with colloidal silver paste. After sputter coating with gold/palladium, the specimens were examined in a JEOL 35C scanning electron microscope at an accelerating voltage of 15 kV and at 86-fold magnification. An acetate sheet bearing a ruled grid was placed over the microscope screen. Each square of the grid had an area of 81 mm², corresponding to 4133 µm² on the specimen. The total area of the specimen, and the area occupied by intimal smooth muscle cells, were determined by counting squares. The extent of smooth muscle cell migration was expressed as the percentage of the total intimal area that was occupied by smooth muscle cells. The results are depicted in Table 1.

**TABLE 1**

| PDGF | | | |
|---|---|---|---|
| | | SMOOTH MUSCLE CELL MIGRATION (%) | |
| TREATMENT | GROUP SIZE | MEAN | STANDARD ERROR |
| PBS | 10 | 4.163 | 1.293 |
| Normal IgG | 8 | 3.513 | 0.806 |
| Anti-PDGF IgG | 8 | 0.737 | 0.310 |

As shown in Table 1, there was no significant difference between PBS and normal IgG groups, but treatment with anti-PDGF IgG reduced the extent of migration by 79.0% (p<0.01).

## Claims (Claims for the following Contracting State(s): AT, BE, CH, LI, DE, DK, FR, GB, GR, IT, LU, MC, NL, SE)

1. Use of an anti-PDGF antibody for preparing a medicament for inhibiting restenosis in a mammal following angioplasty, endarterectomy or bypass surgery.

2. Use according to claim 1 wherein said antibody is a monoclonal antibody.

## Claims (Claims for the following Contracting State(s): ES)

1. Process for preparing a medicament for inhibiting restenosis in a mammal following angioplasty, endarterectomy or bypass surgery, comprising the step of formulating an anti-PDGF antibody into an injectable composition or providing an anti-PDGF antibody in lyophilized form.

2. Process according to claim 1, wherein said antibody is a monoclonal antibody.

## Patentansprüche (Patentansprüche für folgende(n) Vertragsstaat(en): AT, BE, CH, LI, DE, DK, FR, GB, GR, IT, LU, MC, NL, SE)

1. Verwendung eines Anti-PDGF-Antikörpers zur Herstellung eines Arzneimittels zur Hemmung von Restenose nach einer Angioplastie, Endarteriektomie oder Bypass-Operationen bei einem Säuger.

2. Verwendung gemäß Anspruch 1, bei der der Antikörper ein monoklonaler Antikörper ist.

## Patentansprüche (Patentansprüche für folgende(n) Vertragsstaat(en): ES)

1. Verfahren zur Herstellung eines Arzneimittels zur Hemmung von Restenose nach einer Angioplastie, Endarteriektomie oder Bypass-Operationen bei einem Säuger, umfassend den Schritt des Einbringens eines Anti-PDGF-Antikörpers in eine injizierbare Zusammensetzung oder des Bereitstellens eines Anti-PDGF-Antikörpers in lyophilisierter Form.

2. Verfahren gemäß Anspruch 1, bei der der Antikörper ein monoklonaler Antikörper ist.

## Revendications (Revendications pour l'(les) Etat(s) contractant(s) suivant(s): AT, BE, CH, LI, DE, DK, FR, GB, GR, IT, LU, MC, NL, SE)

1. Utilisation d'un anticorps anti-PDGF pour la préparation d'un médicament destiné à inhiber une resténose chez un mammifère après une angioplastie, une endartérectomie ou une intervention chirurgicale de dérivation.

2. Utilisation suivant la revendication 1, dans laquelle l'anticorps est un anticorps monoclonal.

## Revendications (Revendications pour l'(les) Etat(s) contractant(s) suivant(s): ES)

1. Procédé pour la préparation d'un médicament destiné à inhiber une resténose chez un mammifère après une angioplastie, une endartérectomie ou une intervention chirurgicale de dérivation, comprenant l'étape consistant à formuler un anticorps anti-PDGF en une composition injectable ou à mettre un anticorps anti-PDGF sous forme lyophilisée.

2. Procédé suivant la revendication 1, dans lequel l'anticorps est un anticorps monoclonal.
